# EUROPEAN PATENT SPECIFICATION

(11) **EP 0 592 158 B1**
(45) Date of publication and mention of the grant of the patent: **05.12.2001**
(21) Application number: 93307787.7
(22) Date of filing: 30.09.1993
(51) Int. Cl.: C12N 9/38

(54) **Hyperthermostable beta-galactosidases**
Hyperthermostabile beta-Galaktosidase
Beta-galactosidase hyperthermostable

(30) Priority: 05.10.1992 JP 28815692
(43) Date of publication of application: 13.04.1994
(73) Proprietor: TAKARA SHUZO CO. LTD., Fushimi-ku Kyoto 612 (JP)
(72) Inventor: Shimada, Atsushi, Yamatokoriyama-shi, Nara-ken (JP); Odate, Miki, Muko-shi, Kyoto-fu (JP); Hashino, Kimikazu, Takatsuki-shi, Osaka-fu (JP); Asada, Kiyozo, Koga-gun, Shiga-ken (JP); Kato, Ikunoshin, Uji-shi, Kyoto-fu (JP)
(74) Representative: Wakerley, Helen Rachael

(56) References cited:
- EUR. J. BIOCHEM. no. 187 , 1992 , SPRINGER VERLAG, BERLIN, BRD; pages 321 - 328 F.M. PISANI ET AL. 'Thermostable beta-galactosidase from the archaebacterium Sulfolobus solfataricus'

## Description

This invention relates to a β-galactosidase which has a hyperthermostability and is useful in the food industry and a process for producing the same.

β-Galactosidase, which is an enzyme capable of decomposing β-galactoside, has been found out in animals, plants and microorganisms. It is known that this enzyme occurs particularly in bacteria such as *Escherichia coli, Streptococcus lactis, Bacillus subtillis, Streptococcus thermophilus* and *Sulfolobus solfataricus*. This β-galactosidase is applied to the production of low-lactose milk by taking advantage of its ability to hydrolyze lactose into galactose and glucose. It is also applied to the production of galactose or glucose from lactose contained in milk serum which is formed in a large amount in the process of producing cheese.

To apply β-galactosidase to food processing, therefore, it has been demanded to develop an enzyme which can withstand the use at a high temperature from the viewpoint of preventing contamination with microorganisms during the processing and another viewpoint of elevating the solubility of lactose which serves as a substrate. For example, a β-galactosidase originating in *Sulfolobus solfataricus* [see European Journal of Biochemistry, 187, 321 - 328 (1990)] is a thermophilic enzyme having an activity at a temperature of 90°C. However, its activity falls to about 50% after treating at 85°C for 180 minutes.

As mentioned above, a thermophilic and thermostable enzyme has been required in food processing at a high temperature.

### [Summary of the Invention]

In order to solve this problem, the present invention aims at providing a β-galactosidase having an improved thermophilicity and an excellent thermostability.

The first aspect of the invention relates to a hyperthermostable β-galactosidase characterised by having a residual activity of about 80% or above after having been treated at 90°C for 120 minutes and the physicochemical properties recited in claim 1.

The second aspect of the invention relates to a process for producing such a hyperthermostable β-galactosidase characterized by cultivating a bacterium of the genus *Pyrococcus* and recovering the β-galactosidase according to the first aspect of the invention from the culture.

The microorganism to be used in the present invention is not particularly restricted, so long as it produces a hyperthermostable β-galactosidase. For example, strains belonging to the genus *Pyrococcus* exemplified by *Pyrococcus furiosus* DSM 3638 and *Pyrococcus woesei* DSM 3773 are usable therefor. Such a strain is cultivated in an appropriate growth medium and then the hyperthermostable β-galactosidase according to the present invention can be obtained from the cells and the culture.

Now, the present invention will be described by referring to the use of a strain of the genus *Pyrococcus* as an example.

To cultivate a bacterium of the genus *Pyrococcus,* a method usually employed for cultivating a hyperthermostable bacterium may be used. Any nutrient which can be utilized by the employed strain may be added to the medium. For example, starch is usable as a carbon source and trypton and peptone are usable as a nitrogen source. As other nutrients, yeast extract and the like can be used. The medium may contain metal salts such as magnesium salts, sodium salts or iron salts as a trace element. It is advantageous to use artificial seawater for the preparation of the medium, as done in the present invention. The medium is preferably a transparent one free from a solid sulfur element, since such a medium makes it easy to monitor the growth of the cells by measuring the optical density of the culture. The cultivation can be effected either stationarily or under stirring. Alternately, an aeration culture [see Japanese Patent Publication No. 503757/1992] or a dialysis culture [see Appl. Env. Microbiol., 55, 2086 - 2088 (1992)] may be carried out. In general, the cultivation temperature is preferably around 95°C. Usually, a considerably large amount of the β-galactosidase is accumulated in the culture within about 16 hours. It is a matter of course that the cultivation conditions should be determined in such a manner as to achieve the maximum yield of the β-galactosidase depending on the selected strain and the composition of the medium.

The β-galactosidase of the present invention can be recovered by, for example, collecting the cells from the culture broth by centrifuging or filtering and then disrupting the cells. The cell disruption can be effected by, for example, ultrasonic disruption, bead disruption or lytic enzyme treatment. By using these techniques, the β-galactosidase according to the present invention can be extracted from the cells. The enzyme may be extracted by a method capable of giving the highest extraction effect depending on the selected bacterium and thus a crude enzyme solution is obtained. From the crude enzyme solution thus obtained, the β-galactosidase can be isolated by combining techniques commonly employed for purifying enzymes, for example, salting out with ammonium sulfate, ion exchange chromatography, hydrophobic chromatography and gel filtration.

For example, a crude enzyme solution prepared from cultivated cells of *Pyrococcus furiosus* DSM 3638 is chromatographed with a DEAE Toyopearl M650 ion exchanger (mfd. by Tosoh Corporation) to thereby elute an active fraction. The active fraction thus obtained is poured into an HIC-Cartridge Column (mfd. by Bio-Rad) to thereby elute an active fraction. The active fraction thus eluted is poured into a Superose 12 Column (mfd. by Pharmacia) to thereby elute an active fraction. Thus three types of hyperthermostable β-galactosidases (E-I-1, E-I-2 and E-II) can be obtained. The enzymochemical properties of these hyperthermostable β-galactosidases are as follows.

### (1) Action;

Each of E-I-1, E-I-2 and E-II has an action of hydrolyzing lactose into galactose and glucose. Further, each of E-I-1, E-I-2, and E-II has an action of hydrolyzing o-nitrophenyl-β-D-galactopyranoside into o-nitrophenol and galactose.

### (2) Method for determining enzyme activity;

In this specification, determination of enzyme activity was performed by the method described as follows with the exception of the case noted.

In the determination of enzymatic activity, the o-nitrophenyl-β-D-galactopyranoside-hydrolyzing activity of an enzyme can be determined by spectroscopically monitoring o-nitrophenol formed via the hydrolysis of o-nitrophenyl-β-D-galactopyranoside. Namely, 5 µl of the enzyme solution of the present invention is added to a 100 mM phosphate buffer solution (pH 7.3) containing 112 mM of 2-mercaptoethanol and 1 mM of magnesium chloride to thereby give a total volume of 199 µl. Then 1 µl of a dimethyl sulfoxide solution containing 0.4 M of o-nitrophenyl-β-D-galactopyranoside is added thereto. After effecting a reaction at 95°C for 30 minutes, the reaction is ceased by adding 100 µl of 0.1 M sodium carbonate and the absorbance of the reaction mixture at 410 nm is measured to thereby determine the amount of the o-nitrophenol thus formed. One unit of the hyperthermostable β-galactosidase of the present invention is expressed in an amount of the enzyme whereby the absorbance at 410 nm can be increased by 1.0 at 95°C within 1 minute. The enzymes of the present invention, E-I-1, E-I-2 and E-II, each has an activity of decomposing o-nitrophenyl-β-D-galactopyranoside at pH 7.3 at 95°C, as measured above.

Further, the enzymatic activity of the enzyme of the present invention can be determined also by measuring the activity of decomposing lactose. The activity of decomposing lactose is measured by determining the amount of glucose formed by the decomposition of the substrate, as will be described hereinbelow. 5 µl of the enzyme solution is added to 70 µl of a 100 mM phosphate buffer solution (pH 7.3) containing 4 mM of magnesium sulfate. Next, 25 µl of a 2% lactose solution is added thereto and the mixture is reacted at 95°C for 30 minutes. After ceasing the reaction by cooling with ice, the amount of the liberated glucose is determined by using a glucose measurement kit Glucose B-Test Wako (mfd. by Wako Pure Chemical Industries, Ltd.). The enzymes of the present invention, E-I-1, E-I-2 and E-II, each has an activity of decomposing lactose at pH 7.5 at 95°C.

### (3) Optimum temperature:

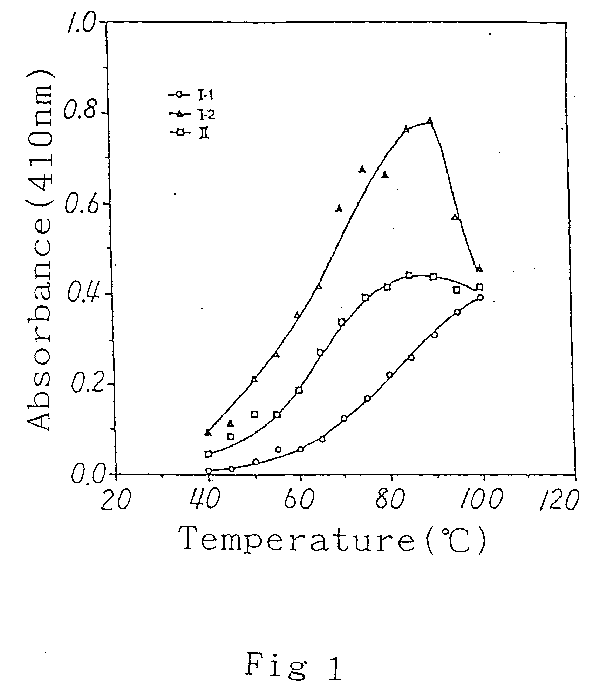

The optimum temperatures of the enzymes of the present invention, E-I-1, E-I-2 and E-II, are measured. With the use of 9.13 mU of E-I-1, 17.25 mU of E-I-2 and 11.35 mU of E-II, the activities of decomposing o-nitrophenyl-β-D-galactopyranoside are determined in accordance with the method described in the above item (2). Fig. 1 shows the results. It is a graph showing the.relationships between the o-nitrophenyl-β-D-galactopyranoside-decomposing activities of the enzymes of the present invention, E-I-1, E-I-2 and E-II, and temperature, wherein the ordinate refers to the absorbance at 410 nm of o-nitrophenol formed by the enzymatic reaction while the abscissa refers to temperature (°C), ○ represents E-I-1, Δ represents E-I-2 and □ represents E-II.

As Fig. 1 shows, each of the enzymes of the present invention, E-I-1, E-I-2 and E-II, is active over a wide range of temperature of from 50 to 100°C at the measurement pH value (7.3). In particular, E-I-1 shows the maximum activity at 100°C. E-I-2 and E-II each shows the optimum temperature within a range of from 85 to 95°C.

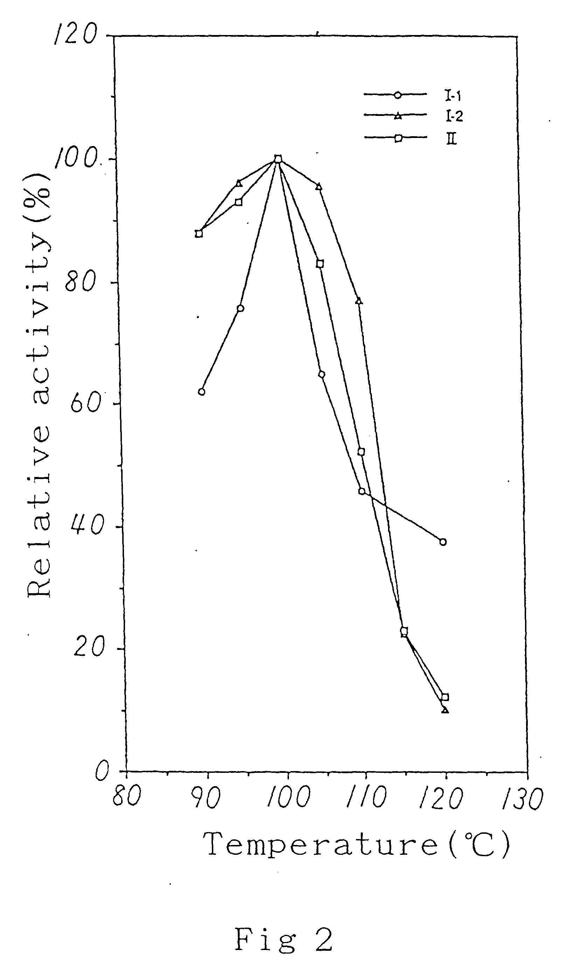

Next, the optimum temperatures of the enzymes of the present invention with the use of lactose as a substrate are examined by using 45.6 mU of E-I-1, 172.5 mU of E-I-2 and 113.56 mU of E-II. The measurement is effected in accordance with the method described in the above item (2) by using a 100 mM glycine - NaOH buffer solution (pH 7.5). Fig. 2 shows the results. It is a graph showing the relationships between the lactose-decomposing activities of the enzymes of the present invention, E-I-1, E-I-2 and E-II, and temperature, wherein the ordinate refers to the relative lactose-decomposing activities of E-I-1, E-I-2 and E-II calculated by taking the lactose-decomposing activity of each enzyme at 100°C as 100% while the abscissa refers to temperature (°C). In Fig. 2, each symbol has the same meaning as the one defined in Fig. 1.

As Fig. 2 shows, E-I-1, among the enzymes of the present invention, shows the maximum activity within a temperature range of from 90 to 105°C while E-I-2 and E-II each shows the maximum activity within a temperature range of from 90 to 110°C.

The reactions are carried out in a water bath (from 40 to 90°C) and in an oil bath (from 90 to 120°C). In particular, the reactions at a temperature exceeding 100°C with the use of lactose as a substrate are performed in a vial provided with a screw cap so as to prevent the reaction mixture from boiling.

The optimum temperatures of the enzymes of the present invention, E-I-1, E-I-2 and E-II, are as follows.
- E-I-1:: 80 - 100°C (using o-nitrophenyl-β-D-galactopyranoside as a substrate).
90 - 105°C (using lactose as a substrate).
- E-I-2:: 75 - 95°C (using o-nitrophenyl-β-D-galacto-pyranoside as a substrate).
90 - 110°C (using lactose as a substrate).
- E-II:: 75 - 95°C (using o-nitrophenyl-β-D-galactopyranoside as a substrate).
90 - 110°C (using lactose as a substrate).

### (4) Optimum pH value:

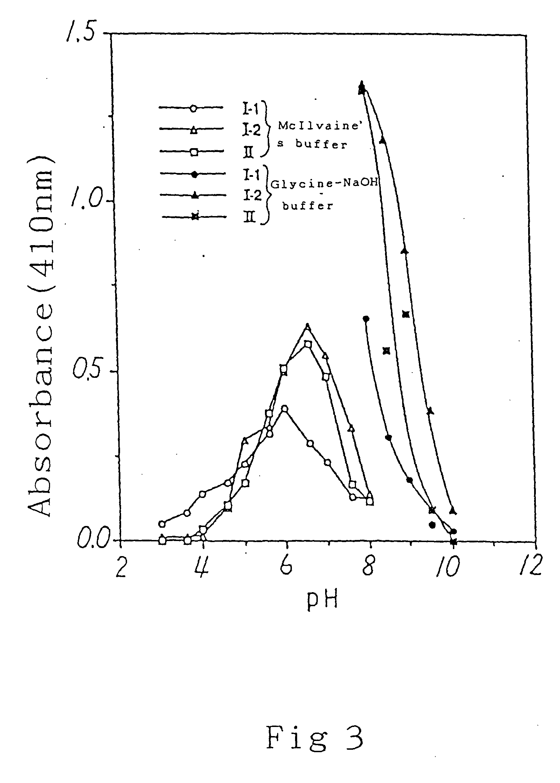

The optimum pH values of the enzymes of the present invention, E-I-1, E-I-2 and E-II, are measured. 9.13 mU of E-I-1, 17.25 mU of E-I-2 and 11.35 mU of E-II are used. The measurement is effected by measuring o-nitrophenyl-β-D-galacto-pyranoside-hydrolyzing activities in accordance with the method described in the above item (2) with the use of McIlvaine's buffer solution (pH 3.0 to 8.0) and a 100 mM glycine -NaOH buffer solution (pH 8.0 to 10.0). Fig. 3 shows the results. It is a graph showing the relationships between the o-nitrophenyl-β-D-galactopyranoside-hydrolyzing activities of the enzymes of the present invention, E-I-1, E-I-2 and E-II, and pH, wherein the ordinate refers to the absorbance at 410 nm of o-nitrophenol formed by the enzymatic reaction while the abscissa refers to pH. In Fig. 3, each symbol has the same meaning as the one defined in Fig. 1 and white marks show the data obtained by using McIlvaine's buffer solution and black ones show the.data obtained by using the glycine sodium buffer solution.

As Fig. 3 shows, E-I-1 is active within a pH range of from 3.0 to 10.0 and shows the maximum activity within a pH range of from 5.6 to 6.6. E-I-2 is active within a pH range of from 3.0 to 10.0 and shows the maximum activity within a pH range of from 6.0 to 7.0. E-II is active within a pH range of from 3.0 to 10.0 and shows the maximum activity within a pH range of from 6.0 to 7.0.

The optimum pH values of the enzymes of the present invention, E-I-1, E-I-2 and E-II, are as follows.
- E-I-1:: pH 5.6 - 6.6
- E-I-2:: pH 6.0 - 7.0.
- E-II:: pH 6.0 - 7.0.

### (5) Thermostability:

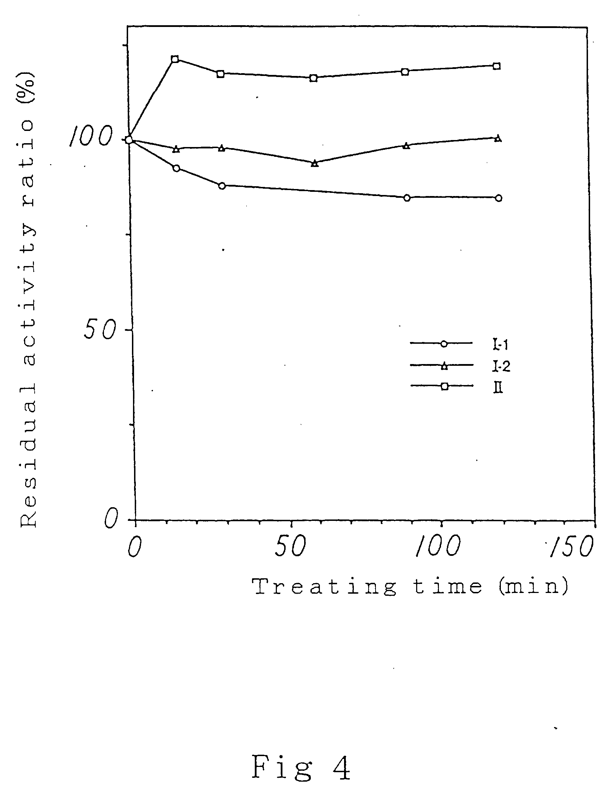

The thermostabilities of the enzymes of the present invention, E-I-1, E-I-2 and E-II, are measured. 9.13 mU of E-I-1, 17.25 mU of E-I-2 and 11.35 mU of E-II are used. The measurement is effected by treating the enzymes in 199 µl of McIlvaine's buffer solution of pH 6.0 (in the case of E-I-1) or McIlvaine's buffer solution of pH 6.6 (in the cases of E-I-2 and E-II) at 90°C for various periods of time and then determining the o-nitrophenyl-β-D-galactopyranoside-hydrolyzing activities in accordance with the method described in the above item (2). Fig. 4 shows the results. It is a graph showing the thermostabilities of the enzymes of the present invention, E-I-1, E-I-2 and E-II, wherein the ordinate refers to the residual activity ratio (%) calculated by taking the activity of each enzyme in an untreated state as 100% while the abscissa refers to treating time (minute). In Fig. 4, each symbol has the same meaning as the one defined in Fig. 1.

As Fig.4 shows, E-I-1 has its enzymatic activity at a level of about 80% even after having been treated at 90°C for 120 minutes. E-I-2 almost completely has its enzymatic activity after having been treated at 90°C for 120 minutes. In the case of E-II, the enzymatic activity is not lowered but rather elevated by about 20% by having been treated at 90°C for 120 minutes.

The thermostabilities of the enzymes of the present invention, E-I-1, E-I-2, E-II, are as follows.
- E-I-1:: About 80% of the activity is observed after having been treated at pH 6.0 at 90°C for 120 minutes. After having been treated for 5 hours, about 50% of the activity is observed.
- E-I-1:: The activity is not lowered but sustained after having been treated at pH 6.6 at 90°C for 120 minutes. After having been treated for 5 hours, about 90% of the activity is observed.
- E-II:: The activity is not lowered but rather elevated to about 120% after having been treated at pH6.6 at 90°C for 120 minutes. After having been treated for 5 hours, about 110 to 120% of the activity is observed.

### (6) pH stability:

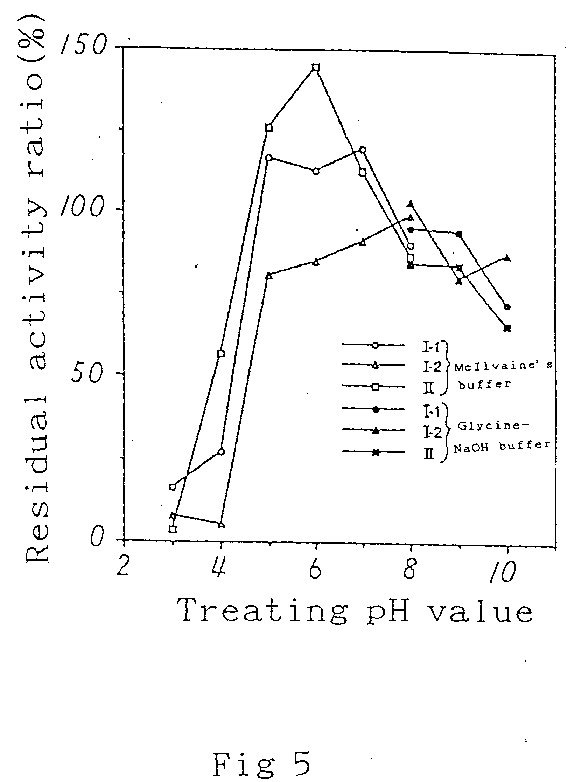

The pH stabilities of the enzymes of the present invention are examined. 9.13 mU of E-I-1, 34.5 mU of E-I-2 and 22.7 mU of E-II are used. McIlvaine's buffer solution is used in a pH range of from 3.0 to 8.0 and a 100 mM glycine - NaOH buffer solution is used in a pH range of from 8.0 to 10.0. To these enzymes are added 9 µl portions of the above-mentioned buffer solutions so as to give a total volume of 10 µl in each case. After having been treated at 90°C for 30 minutes, 199 µl of McIlvaine's buffer solution of pH 6.0 (in the case of E-I-1) or 199 µl of McIlvaine's buffer solution of pH 6.6 (in the cases of E-I-2 and E-II) is added to determine the activity. The activity is determined by measuring the o-nitrophenyl-β-D-galactopyranoside-hydrolyzing activity at pH 7.3 in accordance with the method described in the above item (2). Fig. 5 shows the results. It is a graph showing the thermostabilities of the enzymes of the present invention, E-I-1, E-I-2 and E-II, wherein the ordinate refers to the residual activity ratio (%) calculated by taking the activity of each enzyme in an untreated state as 100% while the abscissa refers to treating pH value. In Fig. 5, each symbol has the same meaning as the one defined in Fig. 3.

As Fig. 5 shows, the enzymes of the present invention, E-I-1, E-I-2 and E-II, each remains stable within a pH range of 5.0 to 10.0 at the measurement temperature (90°C).
- E-I-1:: sustaining its activity after having been treated within a pH range of from 5.0 to 10.0 at 90°C for 30 minutes.
- E-I-2:: sustaining its activity after having been treated within a pH range of from 5.0 to 10.0 at 90°C for 30 minutes.
- E-II:: sustaining its activity after having been treated within a pH range of from 5.0 to 10.0 at 90°C for 30 minutes.

### (7) Substrate specificity:

Substrate specificity of above-mentioned enzymes is able to be determined by using p-nitrophenol-derivatives as shown in Table 1.
The method is shown as follows;
1485 µl of 150mM sodium citrate buffer (pH 5.0) containing above-mentioned enzymes is added to a quartz cuvette for spectrometer.
15µl of 0.1M substrate solution shown in Table 1 is added to the buffer and mixed. Immediately, reaction was followed to completion at 405nm on spectrophotometer. As a blank test, 1485 µl of 150 mM sodium citrate buffer (pH 5.0) not containing enzyme was used, and determination described above was performed. On the test, reaction was performed at 90°C. One unit of enzyme activity was defined as that amount required to catalyze the formation of 1 µmol p-nitrophenol.

According to the method described above, 0.028units of E-I-1, 0.022units of E-I-2, and 0.016units of E-II were used. Hydrolytic activity towards p-nitrophenyl-β-D-glucopyranoside(GlcpβNp), p-nitrophenyl-β-D-galactopyranoside(GalpβNp), p-nitrophenyl-β-D-mannopyranoside(ManpβNp), p-nitrophenyl-β-D-xylopyranoside(XylpβNp), p-nitrophenyl-β-D-fucopyranoside(FucpβNp), p-nitrophenyl-α-D-galactopyranoside(GalpαNp), was determined.

Results were shown in Table 1. The Table shows specific activity of above-mentioned enzymes, E-I-1, E-I-2, and E-II, towards above described substrates and their relative activities.

Above-mentioned enzymes, E-I-1, E-I-2, and E-II, exhibit substrate specifity at 90°C and pH 5.0 as shown in Table 1.

### [Table 1]

**Table 1**

| Specific activity of the β-galactosidases | | | | | | |
|---|---|---|---|---|---|---|
| | E-I-1 | | E-I-2 | | E-II | |
| Substrate | Specific activity | Relative activity | Specific activity | Relative activity | Specific activity | Relative activity |
| | U/mg | % | U/mg | % | U/mg | % |
| GlcpβNp | 0.776 | 100 | 32.3 | 100 | 44.6 | 100 |
| GalpβNp | 0.694 | 89.4 | 12.3 | 38.1 | 19.4 | 43.6 |
| ManpβNp | 0 | 0 | 54.8 | 169.4 | 3.3 | 7.5 |
| XylpβNp | 0 | 0 | 4.2 | 13.1 | 5.4 | 12.0 |
| FucpβNp | 0 | 0 | 0 | 0 | 0 | 0 |
| GalpαNp | 0 | 0 | 0 | 0 | 0 | 0 |

Suitable selection of above-mentioned enzymes, E-I-1, E-I-2, and E-II,is capable of optimal reaction towards a target substrate.

### [Brief Description of the Figures]

[Fig. 1]
   Fig. 1 is a graph showing the relationships between the activities of the enzymes of the present invention with the use of o-nitrophenyl-β-D-galactopyranoside as a substrate and temperature, wherein the ordinate refers to the absorbance at 410 nm of the enzymatic reaction product while the abscissa refers to temperature.
[Fig. 2]
   Fig. 2 is a graph showing the relationships between the activities of the enzymes of the present invention with the use of lactose as a substrate and temperature, wherein the ordinate refers to the relative activity while the abscissa refers to temperature.
[Fig. 3]
   Fig. 3 is a graph showing the relationships between the activities of the enzymes of the present invention and pH, wherein the ordinate refers to the absorbance at 410 nm of the enzymatic reaction product while the abscissa refers to pH.
[Fig. 4]
   Fig. 4 is a graph showing the thermostabilities of the enzymes of the present invention, wherein the ordinate refers to the residual activity ratio while the abscissa refers to treating time.
[Fig. 5]
   Fig. 5 is a graph showing the pH stabilities of the enzymes of the present invention, wherein the ordinate refers to the residual activity ratio while the abscissa refers to treating pH.

### [Examples]

To further illustrate the present invention in greater detail, and not by way of limitation, the following Examples will be given.

### Example 1

2 l of a medium comprising 1% of trypton, 0.5% of yeast extract, 1% of soluble starch, 3.5% of Jamarin S Solid (mfd. by Jamarin Laboratory), 0.5% of Jamarin S Liquid (mfd. by Jamarin Laboratory), 0.003% of MgSO₄, 0.001% of NaCl, 0.0001% of FeSO₄·7H₂O, 0.0001% of CoSO₄, 0.0001% of CaCl₂·7H₂O, 0.0001% of ZnSO₄, 0.1 ppm of CuSO₄·5H₂O, 0.1 ppm of KAl(SO₄)₂, 0.1 ppm of H₃BO₃, 0.1 ppm of Na₂MoO₄·2H₂O and 0.25 ppm of NiCl₂·6H₂O was fed into a 2 l medium bottle and sterilized at 120°C for 20 minutes. After eliminating the dissolved oxygen by blowing nitrogen gas, the medium was inoculated with the above-mentioned strain, which was then stationarily cultivated at 95°C for 16 hours. After the completion of the cultivation, cells were collected by centrifuging. Then these cells were suspended in 30 ml of a 50 mM phosphate buffer solution (pH 7.0) containing 1 mM of EDTA, 1 mM of PMSF and 0.1% of Triton X-100 and disrupted by ultrasonication. This suspension of the disrupted cells was centrifuged at 4°C at 12,000 rpm for 10 minutes and the supernatant was separated for use as a crude enzyme solution. When the content of the enzyme in this crude enzyme solution was measured, it was found out that 169.2 U of β-galactosidase was obtained. The crude enzyme solution thus obtained was adsorbed by a DEAE Toyopearl M650 column (mfd. by Tosoh Corporation) which had previously been equilibrated with a 50 mM phosphate buffer solution (pH 7.0) containing 1 mM of EDTA. After washing this column with the above-mentioned buffer solution and developing with the same buffer solution by linear gradient elution with 0 to 0.5 M sodium chloride, two types of active fractions were eluted. These active fractions were named E-I and E-II fractions in order of elution. The active fractions were separately combined. To the E-I fraction was added 3.6 M ammonium sulfate in such a manner as to give a concentration of 1.2 M. Then the obtained mixture was adsorbed by an HIC-Cartridge Column (mfd. by Bio-Rad) which had been previously equilibrated with a 50 mM phosphate buffer solution (pH 7.0) containing 1.2 mM of ammonium sulfate and 1 mM of EDTA. After washing this column with a 50 mM phosphate buffer solution (pH 7.0) containing 1.2 M of ammonium sulfate and 1 mM of EDTA and developing with a 50 mM phosphate buffer solution (pH 7.0) containing 1 mM of EDTA by linear gradient elution with 1.2 to 0 M ammonium sulfate, active fractions were eluted. These active fractions were combined together, concentrated with Centriflo® CF25 (mfd. by Amicon) and MOLCUT II GC (mfd. by Millipore Corp.) and subjectd to gel filtration by using a Superose 12 Column (mfd. by Pharmacia; 10 x 300 mm) which had been previously equilibrated with a 50 mM phosphate buffer solution (pH 7.2) containing 100 mM of sodium chloride. Thus two active fractions were obtained and named E-I-1 and E-I-2 in order of elution. When the contents of enzymes in the fractions E-I-1 and E-I-2 were measured, it was found out that 1.8 U of E-I-1 and 6.9 U of E-I-2 were obtained.

To the E-II fraction was added 3.6 M ammonium sulfate in such a manner as to give a concentration of 1.2 M. Then the obtained mixture was adsorbed by an HIC-Cartridge Column (mfd. by Bio-Rad) which had been previously equilibrated with a 50 mM phosphate buffer solution (pH 7.0) containing 1.2 M of ammonium sulfate and 1 mM of EDTA. After eluting this column with a 50 mM phosphate buffer solution (pH 7.0) containing 1.2 M of ammonium sulfate and 1 mM of EDTA, active fractions were eluted. These active fractions were combined together, concentrated with MOLCUT II GC (mfd. by Millipore Corp.) and subjectd to gel filtration by using a Superose 12 Column (mfd. by Pharmacia; 10 x 300 mm) which had been previously equilibrated with a 50 mM phosphate buffer solution (pH 7.2) containing 100 mM of sodium chloride. When the content of the enzyme in the fraction E-II was measured, it was found out that 3.4 U of the enzyme was obtained.

The active fractions were detected by measuring the activity of hydrolyzing o-nitrophenyl-β-D-galactopyranoside.

### Example 2

By using the enzyme solutions as obtained in the above Example 1, effects of sodium dodecyl sulfate (SDS) on the enzymatic activities thereof were examined. As a buffer solution, McIlvaine's buffer solution of pH 6.0 was used for E-I-1 while McIlvaine's buffer solution of pH 6.6 was used for E-I-2 and E-II. 9.13 mU of E-I-1, 17.25 mU of E-I-2 and 11.35 mU of E-II were employed. To each enzyme was added the above-mentioned buffer solution containing 112 mM of 2-mercaptoethanol and 1 mM of magnesium chloride in such a manner as to give a total volume of 189 µl. After adding 10 µl of a 10% sodium dodecyl sulfate solution, 1 µl of a 0.4 M o-nitrophenyl-D-galactopyranoside solution was further added thereto to effect a reaction at 90°C for 30 minutes. Then the reaction was ceased by adding 100 µl of a 0.1 M sodium. carbonate solution. The amount of the o-nitrophenol thus formed was measured with the guidance of the absorbance at 410 nm. As the following Table 2 shows, E-II, among the enzymes of the present invention, sustained its activity.

### [Table 2]

**Table 2**

| Influence of sodium dodecyl sulfate in the activities of β-galactosidases | | |
|---|---|---|
| enzymes | residual activities(%) | |
| | SDS(+) | SDS(-) |
| E-I-1 | 0 | 100 |
| E-I-2 | 6.9 | 100 |
| E-II | 126.2 | 100 |

The present invention provides hyperthermostable β-galactosidases and further provides a β-galactosidase which has a hyperthermostability and can exert its action even in the presence of sodium dodecyl sulfate which is a powerful surfactant.

## Claims

1. A hyperthermostable β-galactosidase obtainable from cultivating a bacterium of the genus *Pyroccocus,* having the following properties:
(i) a residual activity about 80% or above after having been treated at 90°C for 120 minutes;
(ii) action and substrate specificity: having an action of hydrolising lactose into galactose and glucose and another action of hydrolysing o-nitrophenyl-β-D-galactopyranoside into o-nitrophenol and galactose;
(iii) optimum temperature: 80-95°C; and
(iv) stable pH range: 5-10

2. A process for producing a hyperthermostable β-galactosidase **characterized by** cultivating a bacterium of the genus *Pyrococcus* and recovering the β-galactosidase as claimed in Claim 1 from the culture.

## Patentansprüche

1. Hyperthermostabile β-Galactosidase erhältlich durch Kultivierung eines Bakteriums der Gattung Pyrococcus, die die folgenden Eigenschaften aufweist:
(i) eine Restaktivität von ungefähr 80 % oder darüber, nachdem sie 120 Minuten lang bei 90 °C behandelt wurde,
(ii) Wirkungs- und Substratspezifität: mit einer Wirkung zum Hydrolysieren von Lactose in Galactose und Glucose und weitere Wirkung zum Hydrolysieren von o-Nitrophenyl-β-D-galactopyranosid in o-Nitrophenol und Galactose,
(iii) optimale Temperatur: 80-95 °C, und
(iv) stabiler pH-Bereich: 5-10.

2. Verfahren zur Herstellung einer hyperthermostabilen β-Galactosidase, **gekennzeichnet durch** Kultivieren eines Bakteriums der Gattung Pyrococcus und Gewinnen der β-Galactosidase wie in Anspruch 1 beansprucht aus der Kultur.

## Revendications

1. β-galactosidase hyperthermostable pouvant être obtenue par culture d'une bactérie du genre *Pyrococcus*, possédant les propriétés suivantes :
(i) activité résiduelle d'environ 80 % ou plus après traitement à 90°C pendant 120 minutes ;
(ii) action et spécificité envers le substrat : action d'hydrolyse du lactose en galactose et glucose et autre action d'hydrolyse du o-nitrophényl-β-D-galactopyrannoside en o-nitrophénol et galactose ;
(iii) température optimale : 80-95°C ; et
(iv) stable dans la plage de pH de : 5-10.

2. Procédé de production d'une β-galactosidase hyperthermostable, **caractérisé en ce qu'**il consiste à cultiver une bactérie du genre *Pyrococcus* et à isoler de la culture la β-galactosidase suivant la revendication 1.
